# EUROPEAN PATENT APPLICATION

(11) **EP 4 574 961 A1**
(43) Date of publication of application: **25.06.2025**
(21) Application number: 23218860.7
(22) Date of filing: 20.12.2023
(51) Int. Cl.: C12M 1/34, C12M 1/36, C12M 1/00, G01N 1/20

(54) **BIOPROCESS ASSEMBLY WITH INTEGRATED FILTRATION AND SAMPLING SYSTEMS**

(71) Applicant: Sartorius Stedim Biotech GmbH, 37079 Göttingen (DE)
(72) Inventor: GRIMM, Christian, 37079 Göttingen (DE); HÖHSE, Marek, 37079 Göttingen (DE)
(74) Representative: Prinz & Partner mbB

(57) **Abstract**

A bioprocess assembly (10) comprises a separation system (12) and a sampling system (14). The sampling system (14) is fluidically connected to the separation system (12) and comprises a sampling membrane (32) such that the separation system (12) and the sampling membrane (32) provide a separate sampling channel (30). The sampling channel (30) comprises at least one fluidic connection (36) to receive a buffer and at least one fluidic connection (51) to a sampling station (50). Further, the sampling channel (30) comprises a counter pressure generating unit (48) and at least one sensor (40), wherein the counter pressure generating unit (48) is configured to adjust the pressure within the sampling channel (30) based on data collected by the at least one sensor (40).

## Description

The invention relates to a bioprocess assembly comprising a separation system and a sampling system.

Spectroscopic techniques are commonly used for process control as they constitute contactless, fast and non-destructive measurements. However, spectroscopic methods are indirect measurement techniques, which means that they require calibration with a sample of known analyte concentration in order to allow for quantitative analyte predictions. Ideally, the sample of known analyte concentration is from the process stream and therefore identical to the measurement sample used for the spectroscopic measurement. In order to achieve this, the sampling points need to be close to the spectroscopic measurement location. Once withdrawn, the samples are analyzed, either on site or remotely, and the obtained analyte concentration is linked to the recorded spectrum.

In single-use bioprocesses, sampling points are mostly located in an intermediate vessel, in a vessel at the end of the process step, or in a vessel at the end of the process. Consequently, the sample only provides the average analyte concentration of the process stream up to the sampling point. However, for the calibration of the spectroscopic technique the sample is required to yield information about the analyte concentration at the specific point in time of the spectroscopic measurement. Therefore, as already indicated above, the sampling system has to be close to the spectroscopic measurement location.

In view of this background, it has been suggested with regard to filtration processes, to integrate the sampling system into a filter system, such as suggested in DE 34 41 249 C2. An alternative solution is provided in EP 2 091 412 B1 and WO 2019/110185 A1, which suggests the use of a dialysis probe for bioreactor applications in order to measure parameters such as nutrients, metabolites, or antibody concentrations.

Apart from that, there is a trend in the industry, especially with respect to continuous or quasi-continuous processes, to monitor critical quality attributes (CQA) of target molecules (antibodies, therapeutic protein, HCP, DNA, residual protein A, etc.) or key performance attributes (KPA) of the process (product concentration, viscosity, etc.) in order to later control critical process parameters CPP (trans membrane pressure, temperature, salt concentration, buffer, flow rate, etc.) which have impact on product quality. These important measurements are most often performed by physical sampling from the process with subsequent (attached at-line or laboratory off-line) analysis in an analyzer, if no in-situ, in-line or on-line measurement with a probe or the like is possible.

However, by repeatedly taking physical samples from the process, the overall process may be disturbed. In particular, taking a sample may change the fluidics, the pressure differences, the flow pattern, etc. A further drawback is that repeatedly taking physical samples results in a significant amount of the processing fluid being withdrawn from the overall process such that a significant amount of expensive medium is lost.

Therefore, it is an objective of the invention to enable an improved sampling process, which provides a more accurate analyte concentration at the spectroscopic measurement point, reduces the process costs and prevents disturbances to the process to the best possible extent.

This objective is solved by a bioprocess assembly comprising a separation system and a sampling system. The sampling system is fluidically connected to the separation system and comprises a sampling membrane such that the separation system and the sampling membrane provide a separate sampling channel. The sampling channel comprises at least one fluidic connection to receive a buffer and at least one fluidic connection to a sampling station. Further, the sampling channel comprises a counter pressure generating unit and at least one sensor, wherein the counter pressure generating unit is configured to adjust the pressure within the sampling channel based on data collected by the at least one sensor. In other words, by having a fluidic connection between the separation system and the sampling system a sample from the separation system may enter the sampling channel through the sampling membrane. In order to only disturb the separation system and the overall bioprocess as little as possible, the sampling channel is provided with a counter pressure generating unit and a sensor to control the transfer of the sample through the membrane.

Since the sampling system includes a pressure control in the form of a counter pressure generating unit and at least one sensor included in the sampling channel, the overall bioprocess, and in particular a separation process taking place in the separation system, is not disturbed, especially in regards to the pressure conditions required for the separation. Furthermore, since the sampling system is fluidically connected to the separation system, it provides an accurate representation of the analyte concentration at the desired measurement point.

A membrane in the sense of the invention can be any layer of material having a selective permeability such as a crossflow filter, a tube filter, a hollow fiber filter, etc.

The counter pressure generating unit comprises a control unit that adjusts the generated pressure after analyzing the data collected by at least one sensor. This way it can be ensured, that the pressure for taking a sample is optimally set without significantly disturbing the pressure required for the separation system.

The sampling system may be integrated into the separation system. In this case, having a counter pressure generating unit becomes even more important, as not having the right pressure for the sampling system may otherwise drastically influence the separation process performed by the separation system. In particular, the counter pressure unit is able to set the pressure within the sampling system based on the pressure, preferably based on the trans membrane pressure, of the separation system.

The buffer is preferably a transport buffer and corresponds to a specially formulated transport medium designed to preserve the sample in a viable state, if necessary, during transport by maintaining osmolarity and pH for up to 56 hours. In particular, when the sampling station comprises an offline analyzer, the sample has to be transported and may not be directly processed, such that the possibility of providing a buffer to the sampling channel is vital.

Preferably, the at least one sensor is a pressure sensor, such that the counter pressure generation unit may generate a counter pressure based on the data provided by the pressure sensor. This simplifies the algorithm used to analyze the sensor data and transmit the impulse to generate a certain counter pressure. Therefore, the sampling environment can be controlled easily without disturbing the overall bioprocess.

Further, the counter pressure generation unit can generate the counter pressure based on data provided by the pressure sensor and data provided by a pressure sensor of the separation system. Therefore, as mentioned already, the counter pressure is set in relation to the pressure of the separation system such that the separation process remains undisturbed while the right pressure for the sampling process is provided in the sampling system.

According to a preferred embodiment, the sampling membrane differs from the membrane used in the separation system in regards to the pore size and/ or the molecular weight cut-off (MWCO). The parameters of the sampling membrane are selected such that the corresponding analyte or analytes are able to pass through the sampling membrane. Regarding materials, the sampling membrane may be made of any of polymer, ceramic, porous metal, etc.

Preferably, the sampling station may be any of an analyzer, a sample collector, a sample routing system and a sample container. This is based on the idea that, depending on the individual setup of the bioprocess assembly, either an in-line or on-line sampling station or an at-line or off-line sampling station can be used. For example, the analyzer may be a classical standard off-line blood analyzer that are for bioreactor samples. Suitable target analytes and/or characteristics to be analyzed in such an analyzer are, for example, glucose, lactate, ammonia, glutamine, glutamate, osmolarity, potassium, sodium, total cell count, and viable cell density. Alternatively, an analyzer that is directly attached to the sampling system may be based on any of the following techniques: liquid chromatography (LC), liquid chromatography mass spectroscopy (LC-MS), (ultra) high performance liquid chromatography ((U)HPLC), gas chromatography (GC), gas chromatography mass spectroscopy (GC-MS), matrix-assisted laser desorption/ionization time-of-flight mass spectroscopy (MALDI-TOF-MS), enzymatic analysis, spectroscopic analysis such as Raman, NIR, MIR, UV/Vis spectroscopy, etc., osmotic strength measurements, pH measurements, conductivity measurements, etc.

According to an embodiment of the invention, the sampling system comprises a fluidic connection to at least one gas source with a septic or sterile connector.

This allows for feeding control gases to the sample, especially in order to prevent cells contained in the sample from dying.

According to a further embodiment of the invention, the sampling system comprises at least one media inlet configured to provide cleaning and/or sanitization fluids to the sampling channel. These media inlets are especially important if the sampling system is not sterile before the first use. Additionally, said media inlets allow to clean the sampling membrane, such that the lifetime of the sampling system may be prolonged.

According to a preferred embodiment of the invention, the bioprocess assembly comprises a valve assembly configured to control the fluidic connections connected to the sampling channel and switch between the different fluidic connections. In other words, the valve assembly that is comprised in the bioprocess assembly allows for selective sampling, such that unless required, no sample is taken from the process stream. That way, the process stream is left intact and no expensive and valuable product is being lost due to continuously taking physical samples from the process stream.

According to an embodiment of the invention, the sampling system comprises a multiplexing unit configured to address different modules in a stack or rack of the bioprocess assembly in parallel. This allows for taking samples from multiple modules at once.

Further, the sampling system may comprise a flow sensor that is configured to determine the sample and/or buffer flow. The data acquired from the flow sensor may also be used by the counter pressure generating unit to adjust the counter pressure accordingly.

According to a preferred embodiment of the invention, the bioprocess assembly comprises a control unit. This control unit is configured to control the sampling process with regards to any of counter pressure, flow speed, valve control, gas mixture and amount, and multiplex regime. That way the entire sampling process can be automated such that the constant presence of an operator in order to take a sample is not required. This drastically lightens the work load and allows for a continuous process control.

According to an embodiment of the invention, a spectroscopic measurement unit is placed right before and/or after the sampling system, such that the sampling time approximately equals the measurement time. This ensures that the sample that is taken is an accurate representation of the measured process stream. In other words, by having the sampling unit right next to or even integrated into the spectroscopic measurement unit, it is assured that the obtained analyte concentration from the sample can be used as a calibration for the spectroscopic measurement.

In this context, "right before and/or after" is to be understood in the sense that no other unit or component of the bioprocess assembly, or a long distance, that could significantly change the properties of the process stream with respect to the intended measurement lies between the sampling system and the spectroscopic measurement unit.

The sampling time refers to the point and time when the sample is taken, which should ideally correspond to the measurement time, which is the time the spectroscopic measurement is made. By having the sampling time and the measurement time be essentially identical or only differ marginally, it is ensured that the sample accurately represents the process medium at the time of the spectroscopic measurement such that the analyte concentration obtained from the sample may be used as a proper calibration means. In other words, the sampling time and measurement time represent the positions of the sampling system and the measurement system and the difference corresponds to the distance between the sampling system and the measurement system. In other words, the composition at the sampling time corresponds to the composition at the spectroscopic measurement time and location. For large distances between the measurement system and the sampling system the sampling system may only represent an average of the analyte concentration contained in the sample.

According to a further embodiment of the invention, the sampling channel is formed by a hollow fiber membrane, which is integrated into a process stream of the separation system. Especially in this embodiment the counter pressure generating unit included in the sampling system is vital since the driving force of a separation system containing a hollow fiber membrane strongly depends on the pressure conditions, particularly the transmembrane pressure.

According to a preferred embodiment of the invention, the sampling system is integrated into a grid module of a modular system for providing the bioprocess assembly. Such a modular system is shown in EP 4 257 669 A1. The sampling system is preferably integrated into a grid module comprising a fluidic connector. The grid module which includes the sampling system, or a neighboring grid module, comprises a separation system and/or the grid module or a neighboring grid module comprises a spectroscopic interface. By having the sampling system integrated into a grid module, the sampling system can easily be integrated into a custom-built or built-to-order bioprocess assembly with the sampling points being selectable depending on the individual setup of the bioprocess assembly. By having the sampling system in the grid module next to, or even integrated into the grid module comprising a separation system and/or the spectroscopic interface, it is assured that the sample accurately represents the analyte concentration of the process stream at the measurement time.

Further features and advantages of the invention will become apparent from the following description and from the accompanying drawings to which reference is made. In the schematic drawings:
- Figure 1 shows a bioprocess assembly according to a first embodiment;
- Figure 2 shows the bioprocess assembly according to a second embodiment;
- Figure 3 shows a sampling system used in the second embodiment shown in figure 2;
- Figure 4 shows the bioprocess assembly according to a third embodiment;
- Figure 5 shows a sampling system used in the third embodiment shown in figure 4; and
- Figure 6 shows the bioprocess assembly according to a fourth embodiment.

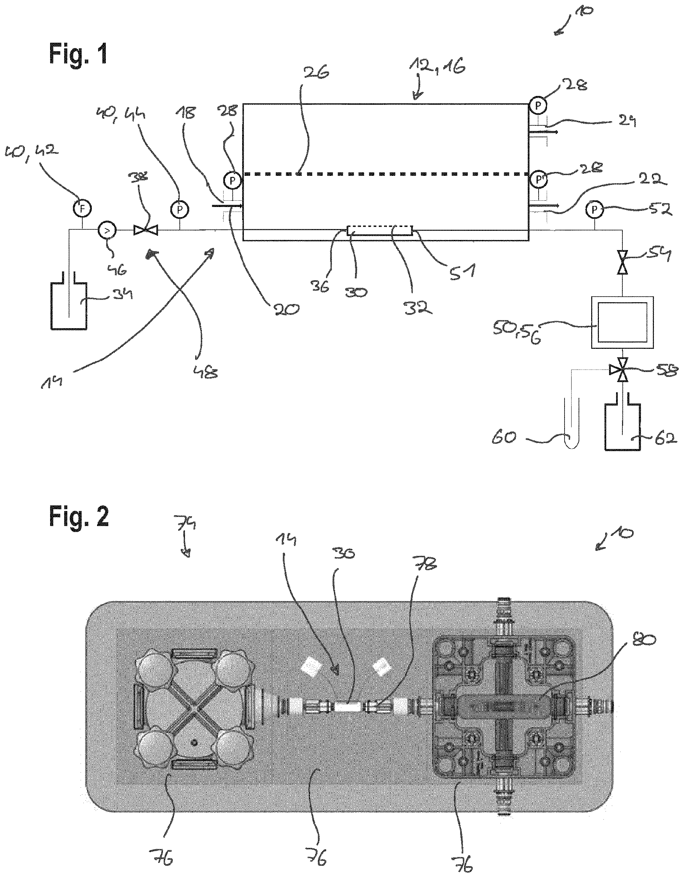

Figure 1 shows a schematic drawing of a bioprocess assembly 10 comprising a separation system 12 and a sampling system 14. The separation system 12 may be used for separation of cells or other substances in a fluid medium by filtration and/or chromatography systems. Possible filtration systems include deep filtration, (alternative) tangential-flow-filtration, sterile filtration, and virus filtration. All these separation systems require stable process conditions in terms of pressure and/or flow rates since their driving force mainly depends on the pressure conditions.

In the embodiment shown in Figure 1, the separation system 12 is a tangential-flow-filtration system 16. The tangential-flow-filtration system 16 comprises an inlet 18 for a process stream 20 as well as a retentate outlet 22 and a permeate outlet 24 which are separated by a membrane 26.

Pressure sensors 28 are associated with each of the fluidic connections, i.e. the inlet 18, the retentate outlet 22, and the permeate outlet 24.

It can be seen in Figure 1 that the sampling system 14 is integrated into the separation system 12. In particular, a sampling channel 30 is placed into the retentate flow.

The sampling channel 30 comprises a sampling membrane 32, which differs from the membrane 26 used in the separation system 12 in regard to the pore size and/or the molecular weight cut-off (MWCO). In particular, the pore size and/or the molecular weight cut-off of the sampling membrane 32 is chosen such that the analyte(s) can pass through the sampling membrane 32.

In particular, when the separation system 12 is used in virus applications, the difference regarding the pore size and/or the molecular weight cut-off of the sampling membrane 32 and the separation membrane 26 are important. The sampling membrane 32 is then chosen such that it blocks virus particles which allows for virus-free sampling. This way, the risk of an operator getting into contact with the virus particles is reduced.

Upstream of the sampling channel 30 and outside of the separation system 12 a tank 34 is located. The tank 34 may contain a buffer, particularly a transport buffer, such that the sampling channel 30 may receive said buffer via a fluidic connection 36 in order to enable transport and/or to preserve the sample.

In Figure 1 it is shown that the fluidic connection 36 between the tank 34 and the sampling channel 30 comprises a control valve 38.

Additionally at least one sensor 40, in particular a flow sensor 42 and a first pressure sensor 44, and a pump 46 are located upstream of the sampling channel 30. The at least one sensor 40 and the pump 46 constitute a counter pressure generating unit 48 that adjusts the pressure within the sampling channel 30 based on data collected by the at least one sensor 40, here the flow sensor 42 and the first pressure sensor 44.

Further, a sampling station 50 is located downstream of the sampling channel 30 and connected to the sampling channel 30 via a further fluidic connection 51. The further fluidic connection 51 comprises a second pressure sensor 52 and a second control valve 54.

The sampling station 50 here corresponds to an analyzer 56. Instead of an analyzer 56 the sampling station 50 may also be a sample collector, a sample routing system, or a sample container. The sampling station 50 is selected based on the parameters that are supposed to be determined and whether or not an in-line or on-line analyzer is available.

The analyzer 56 shown in Figure 1 may, for example, be a classical blood analyzer. Target analytes and/or characteristics to be analyzed in a blood analyzer include glucose, lactate, ammonia, glutamine, glutamate, osmolarity, potassium, sodium, total cell count, and viable cell density.

In continuous or semi-continuous processes it has become common to also monitor critical quality attributes (CQA) of the target molecule such as antibodies, therapeutic protein, host cell proteins, DNA, residual proteins, etc. Furthermore, key performance attributes (KPA) of the process such as product concentration and viscosity are monitored in order to control the critical process parameters (CPP) like the transmembrane pressure, the temperature, the salt concentration, the buffer amount, etc. which have an impact on the product quality.

The measurement of critical quality attributes but also of the critical process parameters and the key performance attributes is often performed with analyzers that include chromatography. Some examples for chromatography are liquid chromatography-mass spectrometry (LC-MS), (ultra) high performance liquid chromatography ((U)HPLC), gas chromatography (GC), gas chromatography-mass spectroscopy (GC-MS), and matrix-assisted laser desorption/ionization time-of-flight mass spectroscopy (MALDI-TOF-MS). Alternatively, enzymatic analyzers, spectroscopic analyzers, i. e. Raman, NIR, MIR, UV/Vis spectroscopy, etc., osmometers, pH probes, conductivity measurement systems, and the like may be used as an analyzer 56.

In the embodiment shown in Figure 1 a 3-way valve 58 is located downstream of the analyzer 56 such that the sample may be received by a sample collector 60 or a waste container 62 after passing through the analyzer 56 depending on the setting of the 3-way valve 58.

As already mentioned above, the sampling station 50 may also be an off-line sampling station such that the sample may be directed into the sample collector 60 or a sample container directly from the sampling channel 30.

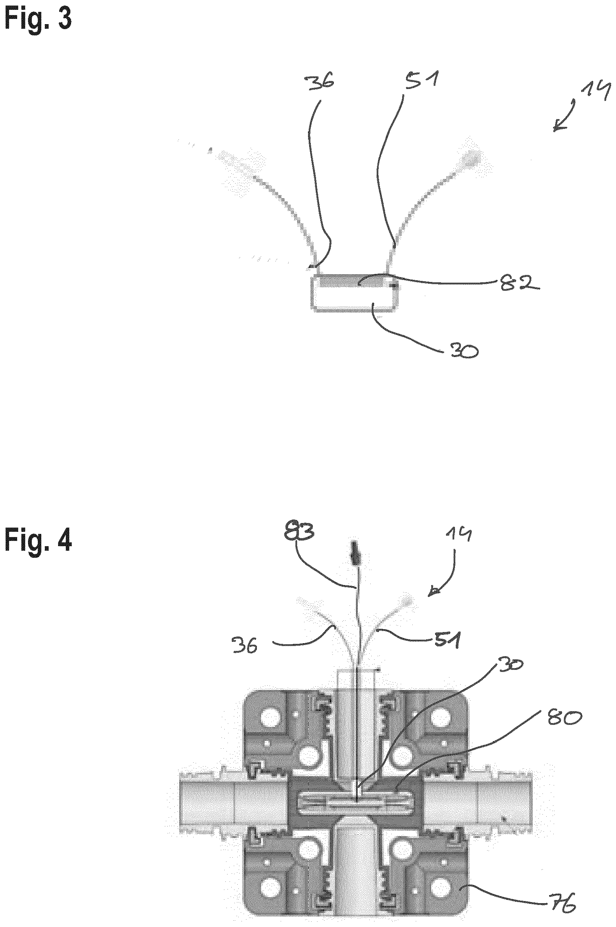

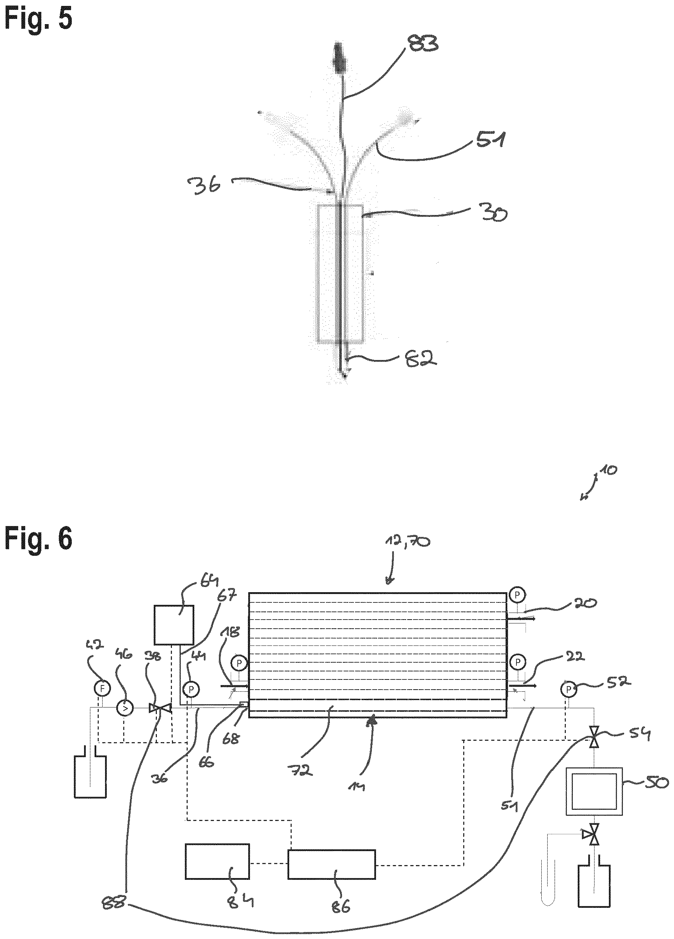

The sampling system 14 may also include a connection to a gas source 64, as shown in Figure 6. The gas source 64 should be connected to the sampling system 14 via an aseptic or sterile connector 66 (see Figures 4 and 5) that constitutes a fluidic connection 67 and provides a gas or a gas mixture to regulate the sampling environment. In particular, the gas source 64 may provide gasses like nitrogen (N₂), carbon dioxide (CO₂), oxygen (O₂) or the like.

Also shown in Figure 6 is a media inlet 68 of the sampling system 14 via which cleaning and/or sanitization fluid can be provided for the sampling channel 30.

The bioprocess assembly 10 shown in Figure 6 further differs from the bioprocess assembly shown in Figure 1 in that the separation system 12 comprises a hollow fiber filtration system 70. Accordingly, the sample channel 30 is formed by a hollow fiber membrane 72 that is integrated into the process stream 20 of the separation system 12, i.e. the hollow fiber filtration system 70.

The bioprocess assembly 10 shown in Figure 2 is a modular system 74 that comprises multiple grid modules 76 arranged on a skid. Such a modular system is disclosed in EP 4 257 669 A1. Each grid module 76 may perform or contribute to a process step of the overall bioprocess, or there may be grid modules 76 which serve as a fluidic connector 78 or manifold.

In Figure 2, the sampling system 14 is integrated into a grid module 76 comprising a fluidic connector 78. Upstream of the sampling system 14 is a grid module 76 comprising a spectroscopic measurement unit 80, which may be connected to a separation system 12 also contained in a grid module 76 (not shown in Figure 2). Since the sampling system 14 is located right next to the spectroscopic measurement unit 80, the sample obtained by the sampling system 14 accurately represents the process stream 20 during the spectroscopic measurement.

The sampling system 14, in particular the sampling channel 30, used in the bioprocess assembly 10 shown in Figure 2 is depicted in more detail in Figure 3. Here, the sampling channel 30 comprises a dialysis membrane 82 to allow for cell free sampling. This means, that cells are not able to cross the dialysis membrane 82. Of course a different kind of sampling membrane 32 may be used instead of the dialysis membrane 82 depending on the type of process.

In the third embodiment of the bioprocess assembly 10 shown in Figure 4 the sampling system 14 is comprised in the same grid module 76 as the spectroscopic measurement unit 80.

The third embodiment of the bioprocess assembly 10 differs from the previous embodiments in that the bioprocess assembly 10 comprises an additional sampling means 83 compared to the embodiment of the bioprocess assembly 10 shown in Figures 2 and 3. The additional sampling system 83 is configured to provide a direct sample, i.e. a sample comprising cells, whereas the sampling system 14 comprises a dialysis membrane 82 in order to allow for cell free sampling.

Figure 5 shows just the sampling system 14 used in the third embodiment. Contrary to the previous sampling system 14, here the sampling channel 30 is perpendicular to the process stream 20 and not aligned with the process stream 20.

In Figure 6 the fourth embodiment of the bioprocess assembly 10 is shown, which also includes a multiplexing unit 84 and a control unit 86.

Preferably, the control unit 86 includes a processing unit and a user interface.

The control unit 86 is configured to control the sampling process with regard to any of the counter pressure, flow speed, valve control, gas mixture and amount and multiplex regime. That means, the multiplexing unit 84 is also controlled by the control unit 86.

The multiplexing unit 84 in turn is able to address different modules of a stack or rack of the bioprocess assembly in parallel such that with the control unit 86 multiple sampling systems 14 included in one or more bioprocess assemblies 10 can be controlled, thus providing a high degree of automation.

The first and second control valves 38, 54 constitute a valve assembly 88 that is able to control the fluidic connections 36, 51 connected to the sampling channel 30. The valve assembly 88 can switch the fluidic connections 36, 51 on and off and completely close the sampling channel 30. Thanks to this switching mechanism it is not necessary to continuously sample the process stream 20 and less volume of the valuable process stream 20 is extracted, thus reducing the overall costs of the process running in the bioprocess assembly 10.

### List of Reference Signs

- 10: bioprocess assembly
- 12: separation system
- 14: sampling system
- 16: tangential-flow-filtration system
- 18: inlet
- 20: process stream
- 22: retentate outlet
- 24: permeate outlet
- 26: membrane
- 28: pressure sensor
- 30: sampling channel
- 32: sampling membrane
- 34: tank
- 36: fluidic connection
- 38: control valve
- 40: sensor
- 42: flow sensor
- 44: pressure sensor
- 46: pump
- 48: counter pressure generating unit
- 50: sampling station
- 51: fluidic connection
- 52: pressure sensor
- 54: valve
- 56: analyzer
- 58: control valve
- 60: sample collector
- 62: waist container
- 64: gas source
- 66: aseptic or sterile connector
- 67: fluidic connection
- 68: media inlet
- 70: hollow fiber filtration system
- 72: hollow fiber membrane
- 74: modular system
- 76: grid module
- 78: fluidic connector
- 80: spectroscopic measurement unit
- 82: dialysis membrane
- 83: sampling means
- 84: multiplexing unit
- 86: control unit
- 88: valve assembly

## Claims

1. A bioprocess assembly (10) comprising a separation system (12) and a sampling system (14), wherein the sampling system (14) is fluidically connected to the separation system (12), wherein the sampling system (14) comprises a sampling membrane (32) such that the separation system (12) and the sampling membrane (32) provide a separate sampling channel (30), wherein the sampling channel (30) comprises at least one fluidic connection (36) to receive a buffer and at least one fluidic connection (51) to a sampling station (50), and wherein the sampling channel (30) comprises a counter pressure generating unit (48) and at least one sensor (40), wherein the counter pressure generating unit (48) is configured to adjust the pressure within the sampling channel (30) based on data collected by the at least one sensor (40).

2. The bioprocess assembly (10) according to claim 1, wherein the at least one sensor (40) is a pressure sensor (44), and wherein the counter pressure generation unit is configured to generate a counter pressure based on the data provided by the pressure sensor (44).

3. The bioprocess assembly (10) according to claim 2, wherein the counter pressure generation unit is configured to generate a counter pressure based on data provided by the pressure sensor (44) and a pressure sensor (28) of the separation system (12).

4. The bioprocess assembly (10) according to any of the preceding claims, wherein the sampling membrane (32) differs from a membrane (26) used in the separation system (12) in regards to the pore size and/or the molecular weight cut-off.

5. The bioprocess assembly (10) according to any of the preceding claims, wherein the sampling station (50) may be any of an analyzer (56), a sample collector (60), a sample routing system and a sample container.

6. The bioprocess assembly (10) according to any of the preceding claims, wherein the sampling system (14) comprises a fluidic connection (67) to at least one gas source (64) with an aseptic or sterile connector (66).

7. The bioprocess assembly (10) according to any of the preceding claims, wherein the sampling system (14) comprises at least one media inlet (68) configured to provide cleaning and/or sanitization fluids to the sampling channel (30).

8. The bioprocess assembly (10) according to any of the preceding claims, wherein the bioprocess assembly (10) comprises a valve assembly (88) configured to control the fluidic connections (36, 51) connected to the sampling channel (30) and switch the different fluidic connections (36, 51) on and off.

9. The bioprocess assembly (10) according to any of the preceding claims, wherein the sampling system (14) comprises a multiplexing unit (84) configured to address different modules in a stack or rack of the bioprocess assembly (10) in parallel.

10. The bioprocess assembly (10) according to any of the preceding claims, wherein the sampling system (14) comprises a flow sensor (42) that is configured to determine the sample and/or buffer flow.

11. The bioprocess assembly (10) according to any of the preceding claims, wherein the bioprocess assembly (10) comprises a control unit (86), which is configured to control the sampling process with regards to any of counter pressure, flow speed, valve control, gas mixture and amount, and multiplex regime.

12. The bioprocess assembly (10) according to any of the preceding claims, wherein a spectroscopic measurement unit (80) is placed right before and/or after the sampling system (14), such that the sampling time approximately equals the measurement time.

13. The bioprocess assembly (10) according to any of the preceding claims, wherein the sampling channel is formed by a hollow fiber membrane (72) which is integrated into a process stream (20) of the separation system (12).

14. The bioprocess assembly (10) according to any of the preceding claims, wherein the sampling system (14) is integrated into a grid module (76) of a modular system (74) for providing the bioprocess assembly (10), preferably into a grid module (76) comprising a fluidic connector (78), wherein the grid module (76) or a neighboring grid module (76) comprises a separation system (12), and/or wherein the grid module (76) or a neighboring grid-module (76) comprises a spectroscopic interface.
